# EUROPEAN PATENT SPECIFICATION

(11) **EP 3 015 478 B1**
(45) Date of publication and mention of the grant of the patent: **11.09.2019**
(21) Application number: 14818010.2
(22) Date of filing: 13.02.2014
(51) Int. Cl.: C08B 15/02, C08B 16/00

(54) **PRODUCTION OF A NOVEL MULTI-PURPOSE CELLULOSIC EXCIPIENT**
HERSTELLUNG EINES NEUARTIGEN CELLULOSISCHEN MEHRZWECKHILFSSTOFFS
PRODUCTION D'UN NOUVEL EXCIPIENT CELLULOSIQUE POLYVALENT

(30) Priority: 27.06.2013 CO 13152949
(43) Date of publication of application: 04.05.2016
(73) Proprietor: Universidad De Antioquia, 050010 Medellin, Antioquia (CO)
(72) Inventor: ROJAS CAMARGO, John, Jairo, 0500100 Medellín Antioquia (CO); LÓPEZ LÓPEZ, Alvin de Jesús, 0500100 Medellín Antioquia (CO)
(74) Representative: Nevant, Marc
(86) International application number: PCT/IB2014/000152
(87) International publication number: WO 2014/207517

(56) References cited:
- EP-A1- 0 460 609
- US-A- 5 410 034
- US-A- 5 410 034
- US-B2- 6 821 531
- US-B2- 6 821 531
- JOHN ROJAS ET AL: "Comparative evaluation of silicified microcrystalline cellulose II as a direct compression vehicle", INTERNATIONAL JOURNAL OF PHARMACEUTICS, ELSEVIER, AMSTERDAM, NL, vol. 416, no. 1, 12 June 2011 (2011-06-12) , pages 120-128, XP028264518, ISSN: 0378-5173, DOI: 10.1016/J.IJPHARM.2011.06.017 [retrieved on 2011-06-17]
- ROJAS: "'Assesment of Processing and Polymorphic Form Effect on the Powder and Tableting Porperties of Mycrocrystalline Celluloses I and II.'", CHEM. PHARM. BULL, vol. 59, no. 5, 1 May 2011 (2011-05-01), pages 603-607, XP055265032,
- ADEL A M ET AL: "Characterization of microcrystalline cellulose prepared from lignocellulosic materials. Part II: Physicochemical properties", CARBOHYDRATE POLYMERS, APPLIED SCIENCE PUBLISHERS, LTD. BARKING, GB, vol. 83, no. 2, 10 January 2011 (2011-01-10), pages 676-687, XP027488150, ISSN: 0144-8617, DOI: 10.1016/J.CARBPOL.2010.08.039 [retrieved on 2010-08-21]
- YUVRAJ P CHAUHAN ET AL: "MICROCRYSTALLINE CELLULOSE FROM COTTON RAGS (WASTE FROM GARMENT AND HOSIERY INDUSTRIES)", INT. J. CHEM. SCI, vol. 7, no. 2, 1 January 2009 (2009-01-01) , pages 681-688, XP055265037,
- ROJAS ET AL.: 'Comparative evaluation of silicified microcrystalline cellulose II as a direct compression vehicle.' INTERNATIONAL JOURNAL OF PHARMACEUTICS vol. 416, 2011, pages 120 - 128, XP028264518
- ROJAS ET AL.: 'Assesment of Processing and Polymorphic Form Effect on the Powder and Tableting Porperties of Mycrocrystalline Celluloses I and II.' CHEM. PHARM. BULL vol. 59, no. 5, 2011, pages 603 - 607, XP055265032
- ADEL ET AL.: 'Characterization of microcrystalline cellulose propared from lignocellulosic materials. PArt. II: Physicochemical properties.' CARBOHYDRATE POLYMERS vol. 83, 2011, pages 676 - 687, XP027488150
- CHAUCHAN ET AL.: 'Microcrystalline cellulose form cotton rags' WASTE FROM GARMENT AND HOSIERY INDUSTRIES vol. 7, no. 2, 2009, pages 681 - 688, XP055265037

## Description

The invention relates to the process for producing a novel multi-purpose agglomerated cellulosic excipient. This excipient replaces many ingredients in the production of tablets due to the fact that it functions as a diluent, a disintegrating agent and an agglutinant, all in the same material. This material is therefore ideal for producing tablets in a dry process such as direct compression and dry granulation, saving production times and costs, especially in humidity- and heat-sensitive drugs. The tablets produced by using said excipient rapidly disintegrate by explosion when they come into contact with water. In addition, said excipient can be used to produce loose and compact make-up powders due to its self-agglutinating and sensory properties on the skin, and, also, functions as a dispersant agent that can be used in the production of semisolid products. It can further be used to produce granules and beads or as a diluent in formulations of hard gelatin capsules, and since it is a source of high fiber, it is a good replacement for insoluble dietary fiber.

This excipient is an agglomerate of a new crystalline form of cellulose (polymorph II) obtained from different sources such as cotton, rice hulls, sugar cane bagasse, sisal bagasse and corncob, α-cellulose or cotton hydrocellulose are preferred sources due to their high performance. This excipient is prepared by treating the cellulose powder with sodium hydroxide within -10 and 10°C in the presence of an inert atmosphere for its polymorphic transformation and activation, followed by the addition of a binder, neutralization, filtration and washing. This is the key and novel step of the invention because the process for producing this crystalline form with special properties is more efficient at these conditions. The resulting pulp contains a humidity within 60 and 90% and it is spray drying or it is subjected to dry sequential granulation in situ. This excipient has a degree of polymerization within 50 and 150, preferably within 50 and 100 with a water retainer capacity of 10 to 20% and preferably 15%. Alternatively, the pulp can be used as an aqueous dispersing agent due to its high affinity with water.

### FIELD OF THE INVENTION

This invention relates to the process of producing a novel multi-purpose agglomerated cellulose excipient of natural origin, which functions as a diluent, binder and/or disintegrating agent to produce solid dosage forms, such as semisolid forms dispersing agent or as a multi-purpose agent for producing loose and compact make-up powders or as a dietary fibre supplement.

### BACKGROUND OF THE INVENTION

Tablets to deliver drugs are prepared by direct compression, wet granulation or dry granulation from a powder mixture of various excipients, which in turn are classified as diluents, binders, lubricants, glidants and disintegrating agents. Disintegrating agents are only used for immediate-release of drugs and for allowing water penetration into the tablet. The diluent is responsible for increasing the volume of the tablet in order to achieve the proper dosage. Binders allow the cohesion of the powder mixture and the formation of the tablet. Lubricants reduce friction within the tablet and the matrix wall, and glidant agents improve the flow of materials.

Commercial microcrystalline cellulose comprises polymorph I (MCCI), and it is an excipient that is used as a diluent and a binder for the production of tablets. Its main advantage is that it allows it to be directly compressed due to its self-binding properties. MCCI is obtained by the partial depolymerization or hydrolysis of higher plant pulp using a diluted mineral acid solution followed by ammonia neutralization, washing and spray drying in order to obtain a white crystalline powder (Pat. No. 2978446). This process removes the amorphous zones and mostly allows leaving crystalline regions. MCCI is commercially available in several references in the size range of about 20-200 µ 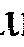 m. MCCI produces hard tablets with the need of adding lubricant and disintegrating agents. This material is more self-lubricating than some abrasive excipients such as lactose, mannitol and dicalcium phosphate. MCCI (e.g., Avicel PH101) has a bulk density from about 0.28 to about 0.37 g/cm³ and settled density from about 0.43 to about 0.48 g/cm³, and it is used as a binder and diluent in formulations made by direct compression, wet and dry granulation in levels of 2-60%.

Moreover, powdered celluloses, are produced by mechanical cellulose disintegration followed by fractionation, filtration, washing and drying (Pat No. 4.269.859; Pat No. 4.438.263). Avicel® and Prosolv® are the most important commercial products of microcrystalline cellulose (MCCI). There is also a low crystalline cellulose obtained by reacting cellulose with phosphoric acid at room temperature for one hour followed by heating within 45 and 75°C for 2-10.5 h, and precipitation in water (Pat. No. 5.417.984).

Cellulose alkaline treatments or mercerization have been used in the textile and paper industry to produce cellulose ethers, esters, xanthates, etc. and to enhance its reactivity with other agents. This process is often used in the textile industry in order to incorporate dye. In the prior art there are also some other processes related, as the one disclosed in US 2002041961, which relates to a process for the manufacture of a composition having a low average degree of polymerization values, wherein said composition is useful for making lyocell fibres, having a high hemicellulose content, a low copper number and including cellulose that has a low average degree of polymerization (DP) and a narrow molecular weight distribution.

The process described therein comprises the steps of contacting an alkaline pulp having a high hemicellulose content of at least 7%, with an oxidant sufficient to reduce the average degree of polymerization to about 200 and 1100 without substantially reducing the hemicellulose content or increasing the copper number of the pulp. The oxidant used in the process disclosed herein may comprise at least one member of the group consisting of a chemical with a peroxide group, oxygen, chlorine dioxide, ozone and combinations thereof, wherein the reduction in the average degree of polymerization of the cellulose occurs in the presence of a ratio of magnesium to transition metals of less than about 50%.

Spanish patent ES 2174830 relates to the preparation of microcrystalline cellulose products (crystallinity of at least 78%) via enzymatic hydrolysis of certain cellulosic materials, wherein this process is particularly useful for converting a cellulosic material to crystalline cellulose comprising subjecting the cellulosic material to hydrolysis using a cellulase enzyme at a temperature of 35-75°C, and at a pH of from 2.0 to 4.0, for a time sufficient to form crystalline cellulose with a degree of polymerization from 800 down to the level-off degree of polymerization (LODP) of the starting material, characterised in that the cellulosic material is a wetlap pulp cellulosic material, that has never been dried. In one embodiment, the starting cellulosic material can be alpha cellulose obtained from fibrous plant material, such as a dissolving grade of wood pulp.

International patent application WO 2010/131088 discloses a method for preparing microcrystalline cellulose, comprising a prior step of compaction of the cellulose before degradation of the glucose chains and obtaining the suitable degree of polymerization.

Said method allows a considerable reduction in the consumption of energy, water and possible chemicals, which are used for reducing the degree of polymerization. Furthermore, the microcrystalline cellulose obtained can be used as a pharmaceutical excipient in tablets, and presents disintegration features comparable to those of the microcrystalline cellulose obtained by a spraying process. Finally, the disclosed method comprises a step of isolating the microcrystalline cellulose obtained in the previous step.

On the other hand, US 2012223165 discloses a method for obtaining microcrystalline cellulose from residues derived from acid delinting of cottonseed, characterized in that the raw material comprises acid residues arising from the cottonseed cleaning process, which are subjected to the stages of neutralization of such residues; cleaning and purification of the acid linter residue;
The chemical neutralization treatment is carried out by means of the action of alkalis and water washing and subsequent thickening by means of filters. Such residues include some fibrils and cascarilla, with a marked presence of vegetal elements typical of the seed. Therefore, after washing and subsequent thickening, the material is subject to the stages of bleaching by means of chemical agents, washing and thickening, drying and dry grinding for reducing particle size.

In this regard, it is clear that the pharmaceutical industry is in constant search of excipients for direct compression, rapidly disintegrating wet and dry granulation, useful for various applications such as sublingual and pediatric administration, which do not require many additional ingredients. It is also important to find substituents of common minerals used in the formulation of loose and compact powders that come from renewable vegetable sources as well as from good dietary fiber sources.

It is a purpose of the present application to produce a novel multi-purpose cellulose excipient that functions as a binder, diluent and disintegrating agent in the design of solid dosage forms, besides being self-binding, to improve the flow and to facilitate the incorporation of pigments and adhesion of loose and compact make-up powders.

It is another purpose to provide a novel multi-purpose cellulose excipient from natural cellulose residues that simultaneously function as diluent, binder and disintegrating agent providing rapidly disintegration of tablets. This excipient is ideal in technologies of wet and dry granulation and direct compression due to its multifunctional properties.

It is further a purpose of the present application to produce a self-agglutinating excipient with good flow, adhesiveness, extensibility and coating for the production of loose and compact make-up powders.

Another purpose of the present application is to provide an excipient having good water absorption that functions as a dispersing agent of semisolids and as an oil absorption modulator and as a source of dietary fibre.

It is further a purpose of the present application to provide a multifunctional excipient being silky, having good absorption of ultraviolet radiation and easily mixed with pigments for the production of powders and sunscreen agents in cosmetic semisolids.

### DESCRIPTION OF THE FIGURES:

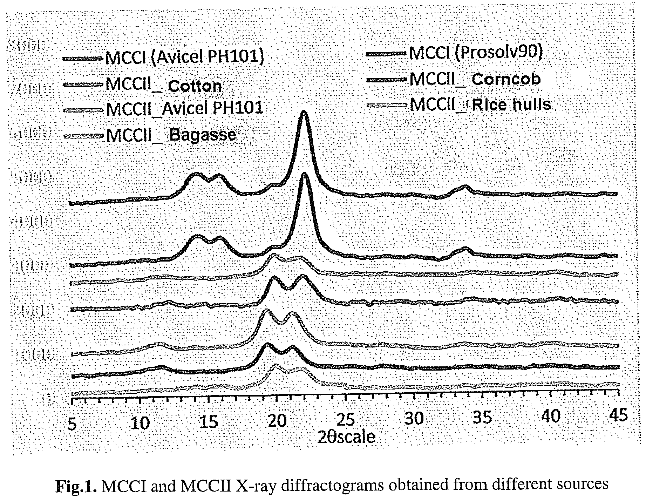

**Figure 1****.** MCCI and MCCII X-ray diffractograms obtained from different sources.
**Figure 2****.** Photomicrographs of the novel agglomerated excipient.
**Figure 3****.** Dissolution profiles of verapamil.HCI tablets made with the agglomerated excipient.
**Figure 4****.** Dissolution Profiles of spironolactone tablets made with the agglomerated excipient.
**Figure 5****.** Variation of the absolute viscosity of the agglomerated excipient in semisolid formulations containing different plasticizers (dibutyl sebacate, polypropylene and dimethicone).
**Figure 6****.** Variation of the hydrophobicity of compact make-up powders determined as the contact angle.

### BRIEF DESCRIPTION OF THE INVENTION

This invention is based on a unique and efficient process for producing an agglomerated microcrystalline cellulose excipient II from agro-industrial residues. This invention involves the cellulose alkaline treatment obtained from agro-industrial residues at low temperatures and in an inert atmosphere to form pulp cellulose II, which when activated in an alkaline medium is dispersed with a binder (talc, magnesium carbonate, silicon dioxide amorphous, bentonite, mica, titanium dioxide, zinc dioxide, kaolin and zinc stearate) using a high intensity homogenizer that, after neutralization and washing, is subject to sequential granulation with drying in situ or spray drying.

The preferred starting material is α-cellulose extracted from cotton, sugar cane bagasse, sisal, rice hulls and corncobs. Other sources of cellulose include commercially available cellulose excipients such as regenerated cellulose, microfibrillated cellulose, powdered cellulose, etc.

The powdered and dried starting material (agro-industrial residues) is immersed in an aqueous sodium hydroxide solution for a period from about 0.5 to about 72 hours within - 10 and 10°C in an inert atmosphere. The preferred soaking time is about 4 to 48 hours, depending on batch size. The concentration of sodium hydroxide may be greater than 3N. Preferably, the concentration of the base ranges within 5 and 7 N. Other inorganic hydroxides such as KOH and Ca(OH)₂ produce an incomplete polymorph conversion.

It is important to use the sodium hydroxide aqueous solution in sufficient amounts as to be fully absorbed and to convert starting cellulose into a pasty or inflated mass. In this regard, a ratio by solid weight to solution of 1:10 is used. A periodically or constantly stirring of the sodium hydroxide solution during the mixing of cellulose powder for the preparation of a homogeneous gel can be used. Particularly, this gel is for dispersing a binder in an amount within 2.5-50% of the cellulose in a high intensity homogenizer. The next step in the process is to regenerate the cellulose from the cellulose gel by neutralization with a mineral acid. The pulp is filtered and washed with water until obtaining a neutral pH. The resulting pulp contains 1-50% by weight of microcrystalline cellulose II (MCCII), preferably from 10 to 30%.

This pulp of regenerated cellulose can be used to produce colloids, semisolids, suspensions, etc. Furthermore, the regenerated cellulose pulp can be dried by spray or by sequential granulation in situ for producing cellulose beads or particles, which may be used as a contributory for producing modified release tablets, pesticides, herbicides and absorbents. The preferred method is the sequential granulation in situ.

The degree of polymerization (DP), which is the average number of glucose units of the cellulose, after hydrolysis, is 50-150, depending on the source used, but a DP within 50 and 100 is preferred. This range is achieved with treatment with NaOH within 5-7N. The resulting dried material of spray drying or granulation sequential granulation functions as a binder, diluent and disintegrating agent, due to the greater affinity of the novel cellulose excipient with water molecules.

For pharmaceutical purposes, the content of binder added to the cellulose gel is ranging from about 2.5 to about 50% with particle sizes preferably from about 500 nm and 30 µm, also the binder surface area ranging from about 0.5 to about 5 m²/g, preferably from 2 to 4 m²/g.

The new excipient obtained by adding the binder to the cellulose gel can be used for producing immediate-release tablets of verapamil.HCl and spironolactone by direct compression, wet and dry granulation. This excipient can also be adapted for obtaining a modified dosage form by combining it when it is in an alkaline state with shellac, chitin, chitosan or derivative methacrylic acid polymers. Both the new crystalline form and the binder improve the functional properties of the microcrystalline cellulose I like the rapid disintegration as well as retaining its good mechanical and silky properties, which makes it ideal for producing loose compact make-up powders. It is also useful as a source of dietary fiber. This novel excipient, in a pulp form, may also be used to manufacture the semisolids, due to its high viscosity at high pHs and formulations containing high salt concentrations, which does not happen with commercially viscosifying agents such as carbopol, xanthan gum, sodium alginate and carboxymethyl cellulose.

### DETAILED DESCRIPTION OF THE INVENTION

The starting material is α-cellulose extracted from cotton, sugar cane bagasse, sisal, rice hulls and corncobs. Other sources of cellulose include any higher plant and commercially available cellulose excipients. Before converting cellulose I into cellulose II and the agglomeration process of the novel excipient, which is the purpose of the invention, the following must be taken into account:
If the starting source contains hemicelluloses and lignin, it must be subjected to a preliminary step of hydrolysis with NaOH at 80°C for 3 h for removing lignin and hemicelluloses. The resulting material is washed, filtered and dried and subjected to reduction with sodium hypochlorite at room temperature for 6-24h for clarification. Then it is neutralized, filtered and washed until obtaining a pulp.

If the starting source is cotton, the cellulose fibers are treated with a mineral acid diluted at 100-105°C for 3 h or during a reasonable period until a dispersion composed of a fine powder in suspension is formed. Suitable mineral acids for this purpose include hydrochloric acid, sulfuric acid, nitric acid, etc., in a concentration from about 0.5 to about 2.5N, but HCl to 1.5N is preferred. The solids content of the resulting aqueous pulp ranges from about 1 to about 60% and preferably from about 10 to about 30% w/w. This material is then subjected to neutralization, filtration and washing.

Obtained hydrocellulose is then immersed in an aqueous sodium hydroxide solution for a period from about 0.5 to about 72 h at a temperature within -10 and 10°C in an inert atmosphere (e.g. N₂, CO₂, etc.). The preferred soaking time is about 4-48 h. Sodium hydroxide is used in concentrations greater than 3 N, preferably from about 5 to about 7 N. This process converts the cellulose into a colloidal complex with sodium making it very reactive, increasing the space within polymer chains and rearranging it. The preferred ratio of the solid and NaOH solution is 1:10 and is accompanied by periodic stirring for achieving homogeneity. This process not only induces the crystalline transformation of the material, but also removes all traces of lignin or hemicellulose present in the starting material.

After this treatment, the cellulose gel is diluted to a concentration of 5-10% w/w and a binder (e.g., MgCO₃, kaolin, mica, amorphous silicon dioxide, talc, bentonite, titanium dioxide, zinc dioxide, zinc stearate, chitosan, shellac and methacrylic acid derivatives) in proportions of 2.5-50% w/w is added, by vigorously stirring within 10,000 and 20,000 rpm for 10-15 min to facilitate the incorporation of the binder within the cellulose chains, which in turn are in a transitional amorphous state. This binder gives interfacial properties that improve the functionality and reduce the phenomenon of hornification during the drying step. The dispersion is then neutralized with a mineral acid to regenerate the cellulose in the crystalline form II in close association with the binder, and is washed until obtaining a conductivity <20 µS/cm.

The novel excipient in the form of a pulp, alone or with other viscosifying agents such as sodium carboxymethylcellulose, hydroxyethylcellulose, carbopol, wetting agents, and emulsifiers such as poloxamers, polysorbates, sodium lauryl sulfate, etc. may be used as an auxiliary in the production of colloids, semisolids, suspensions, creams and gels.

Semisolid cosmetic preparations comprise ingredients such as preservatives, perfumes, antifoams, dyes, pigments, surfactants, wetting agents and viscosifying agents. Examples of semisolid cosmetic formulations of this invention comprise from about 0.5 to about 90% w/w of excipient, but 20-50% is preferred.

If it is desired to obtain the excipient as a dry material, the prior pasty material is spraydried or dry in-situ until obtaining a fine agglomerate.

The degree of polymerization (DP) of resulting material from this process is from about 50 to about 150, but a DP within 50 and 100, which is controlled by the alkali treatment, is preferred.

The resulting dry excipient is used as a multi-purpose excipient, i.e., as a diluent, binder and disintegrating agent. Agglutination occurs by its capability to form dried hydrogen bonds and to disintegrate by the antiparallel orientation of the cellulose chains, which makes the 6 carbon hydroxyl group to be more accessible to water. It can also be used to produce modified-release tablets, absorbents, pesticides and herbicides. This powder was used in direct compression processes, wet granulation and dry granulation to produce immediate-release tablets. This excipient can be adapted for obtaining a modified dosage form, namely, for the release at a constant rate while maintaining the therapeutic effect without achieving toxic effects for 6-24 h.

The surface area of the novel excipient is 0.5-5 m²/g and its particle size is <60 µm and produces tablets with rapid disintegration (<0.8 min), high compressibility (>28%) and fast flow (>3g/s). Rapid disintegration of tablets is due to the high affinity for water of this polymorph of cellulose compared to commercial cellulose such as Avicel® and Prosolv® used in direct compression processes, wet and dry granulation (Table 1).

The novel excipient is insoluble in water and its particle size is controlled directly by the depolymerization and the dry granulation process in situ or spray drying it is subjected to.

The sequential wet granulation process produces granules that are dried in the same equipment. In this agglomeration, the wet mass is passed through four different opened net # 24, 40, 100 and 400 respectively, while simultaneously drying at the same equipment. This drying technique allows the binder to be distributed around and/or inside the cellulose particles giving it special properties. If necessary, additives can be added to the wet mass such as surfactants and plasticizers.

An alternative process is spray drying, wherein the cellulose aqueous dispersion containing binder is subjected to atomization in the presence of hot air (140-200°C). Droplets contacting hot air evaporate and form agglomerated particles. The hot air carries the cyclone formed particles where they are collected as dust.

| **Table 1.** Physical features of MCCII: Binder (75:25) for pharmaceutical purposes | | | | | | | |
|---|---|---|---|---|---|---|---|
| **Used binder or commercial product** | **Compressibility (%)** | **Bulk density (g/cm³)** | **Particle size (µm)** | **Porosity (%)** | **Compactness (KPa)** | **Flow (g/s)** | **Disintegration (min)** |
| Kaolin | 35±1 | 0.28±0.03 | 39.6±5.3 | 83±2 | 212±45 | 3.4±0.2 | 0.68±0.02 |
| Talc | 28±4 | 0.53±0.04 | 30.4±3.5 | 62±3 | 100±34 | 7.5±0.8 | 0.27±0.01 |
| MgCO³ | 37±3 | 0.23±0.06 | 60.4±7.4 | 86±5 | 197±26 | 3.2±0.10 | 0.80±0.02 |
| Avicel®PH101 | 27±2 | 0.35±0.02 | 41.9±0.8 | 76±3 | 420±15 | 0.17±0.01 | 320±45 |
| Prosolv®90 | 22±1 | 0.28±0.02 | 110±9 | 82±2 | 425±23 | 3.0±0.03 | 376±56 |

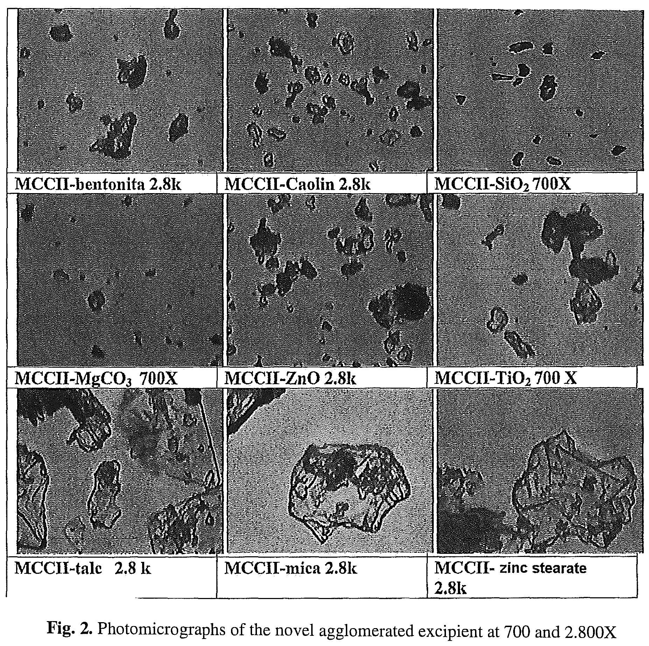

Produced dry excipient does no longer have a fibrous form but is more regular. This is the reason why this material is denser, has improved flow and more compactable than the fibres of the source of the original residue. The binder may be incorporated in or out of the cellulose particles (Fig. 2). The wet granulation process by sequential drying or spray drying allows a physical interaction, in particular by the formation of hydrogen bonds and van der Waals forces that are formed in the polymorph and agglomeration conversion processes. The agglomeration process is quite different from the physical mixture of the components that always entails the phenomenon of segregation and bad flow.

The average particle size of the excipient of this invention ranges from 30-60 µm if a pharmaceutical use is required. The technology can closely control the size and distribution of particle achieving sizes under 30 µm if a cosmetic use is required. For example, humidity, pore size and atomizing nozzles can be controlled within required ranges depending on the final use.

This new excipient can be used to formulate drugs in wet granulation, direct compression or double compression operations. In wet granulation, the excipient is mixed with the drug and subjected to granulation without the need of adding the only water binder that is added until the wet mass acquires a pasty consistency (15-40%). This material is then passed through a # 24-40 mesh, dried at any drying equipment and finally subjected to dry screening through a # 40-100 mesh.

In the pharmaceutical industry, more than 70% of the manufacturing processes are made by wet granulation. However, there are many drugs that for their physicochemical instability (e.g., humidity and heat) cannot be made in this technology. Fortunately, there is a dry granulation process consisting on forming granules without the need of using a liquid dispersion. Thus, drugs or excipients with good cohesive properties are required in dry granulation, and the addition of a dry binder to the formulation to facilitate granule formation is required. Dry granulation requires prior densification and compaction of the powders by two classical methods. The first one also called double compression involves forming ingots in a special tableting machine and the second method requires a roller compactor.

As the dry granulation by double compression produces granules densification at low pressures, formed ingots are easily broken for producing the desired (agglomerated) granules. Drug dissolution rates compared to the wet granulation are increased with the dry granulation.

This excipient has self-lubricating properties; however, lubricants such as magnesium stearate can be added depending on the drug. The mixture of components can be tableted at compression pressures within 50 to 180 MPa.

In certain cases, the novel excipient can be used for modified-release applications of 6-24 h. In this case, the excipient has been agglomerated with shellac, chitosan, acrylic acid derivatives etc. Thus, excipient matrices and the drug may be prepared by direct compression, or simply by adding a binder to form granules followed by compression. Finally, the novel excipient can be used for extrusion and spheronization operations followed by encapsulation. Another application of this excipient is the filling of hard capsules by mixing the drug and the novel excipient, followed by filling capsules with granules made from this material.

The properties of the agglomerates of this excipient for cosmetic purposes are shown in Table 2. In this case, the novel excipient is compared to commercial products as Covabead® and Sericite®.

The particle size for cosmetic application was <30 µm and the most comparable product to Covabead® was the agglomerated excipient with mica, magnesium carbonate, titanium dioxide or zinc dioxide. Similarly, the most comparable excipient in size to Sericite® was the agglomerated with zinc stearate, kaolin or silicon dioxide.

The physical interaction of the binder with cellulose is the one that makes it possible to acquire the properties of silkiness, spreading or coating power and adhesiveness desirable for cosmetic purposes (Table 2).

| **Table 2.** MCCII properties:Aglomerated (75:25) for cosmetic purposes | | | | | | | | |
|---|---|---|---|---|---|---|---|---|
| **used agglomerate/commercial product** | **porosity (%)** | **diameter (µm)** | **compressibility (%)** | **flow (g/s)** | **compactness (N)** | **UV absortion (nm⁻¹)** | **extensibility (%)** | **hydrophobicity/contact angle (°)** |
| SiO₂ amorphous | 79.5±2.5 | 18.6±5.7 | 36.4±5.2 | 4.3±0.2 | 227.9±12.6 | 69.2 | 225.3 | 28.9 |
| zinc dioxide | 78.8±1.4 | 7.3±1.2 | 39±4.7 | 4.2±0.7 | 264.8±5.1 | 419.3 | 115.4 | 19.5 |
| kaolin | 82.9±2.4 | 19.5±3.4 | 41.3±2.7 | 2.7±0.3 | 169.1±4.6 | 304 | 111 | 30 |
| Talc | 77.1±1.5 | 26.4±7.7 | 41.6±3.2 | 4.1±0.2 | 45.7±2.6 | 389.9 | 92.1 | 47.5 |
| Titanium dioxide | 77.7±4.5 | 1.1±0.4 | 41.4±6.5 | 4.2±0.6 | 68.2±2.6 | 438.9 | 125.2 | 25.5 |
| Zinc stearate | 75.4±4.6 | 18.5±2.6 | 37.9±6.4 | 3.0±0.6 | 35.6±4.6 | 264.7 | 87.4 | 110.6 |
| Magnesium carbonate | 83.4±4.6 | 6.7±0.8 | 38.7±3.0 | 4.2±0.2 | 104.6±11.4 | 554.5 | 123.5 | 20.4 |
| Bentonite | 82.3±2.1 | 29.5±9.2 | 44.0±4.0 | 4.2±0.5 | 134.7±18.7 | 361.9 | 61.5 | 27 |
| Mica | 82.4±3.6 | 7.2±1.9 | 40.7±2.0 | 3.9±0.3 | 30.1±7.8 | 388.5 | 23.5 | 5.4 |
| Covabead® | 82.6 | 5.3±1.7 | 45.4 | 2.9±0.3 | 2.1±0 | 354.7 | 405 | 96.5 |
| Sericite®. | 86.5 | 18.5±2.5 | 55.8 | 7.0±2.4 | 3.5±0 | 88.2 | 45.7 | 48.7 |

Due to the morphology of the novel cellulose excipient and its capability to form hydrogen bonds, it is 30 times more compactable than Covabead® and Sericite®. Furthermore, the novel excipient has better ultraviolet radiation absorption properties than the Sericite® and UV radiation absorption comparable to Covabead®. This makes that this novel excipient may act as an opacifying and an auxiliary agent in the preparation of sunscreen lotions. Regarding extensibility or coating power in most cases, it is comparable or better than Sericite®. Furthermore, the novel agglomerated excipient is less hydrophobic than commercial products except agglomerated excipients with talc or zinc stearate whose adhesiveness in the skin was comparable.

### EXAMPLES

The examples below illustrate various aspects of the invention and are not limited only to the claims. The examples illustrate various levels of agglomeration of cellulose II.

### Example 1: Binderless Cellulose II

### Example 2: Cellulose II-Binder (97.5: 2.5)

### Production of agglomerated excipient

**Method 1:** the hydrolyzed and clarified wet cellulose pulp is mixed and alkalinized with NaOH (7N) in the cold (4°C) and under an inert atmosphere for 24 h at 4°C with 2.5% of binder (e.g., talc, kaolin) on a dry basis, in a high intensity homogenizer or colloid mill for 10 min and at 15,000 rpm to form a 10% dispersion. The mixture was then filtered under vacuo and the homogeneous resulting pulp was passed through a sequential drying oscillating granulator equipped with mesh openings of 3350, 2000, 711, 425 and 150 µm when the humidity content was reduced in situ to 60, 50, 40, 30 and 20%, respectively. The powder obtained was dried in a tray oven for 3h at 100°C. The particle size obtained was between 30 and 60 µm.

**Method 2:** About 500 g of hydrolyzed and clarified wet cellulose pulp with a humidity content of 60-80% was taken and alkalinized with NaOH (7N) in the cold and under an inert atmosphere for 24 h at 4°C, and water was added until obtaining a dispersion of solids at 5%.

A binder (e.g., talc, kaolin) equivalent to 2.5% by weight was added and the mixture was subjected to homogenization in a colloid mill or homogenizer for 10 minutes at 15,000 rpm, and then to neutralization, filtration and washing. The pulp obtained was diluted to form a solid content of 3% and passed through a spray dryer Bucchi (B-290, Zurich, Switzerland). The operating conditions were: injection temperature 194°C, outlet temperature 66°C, drying rate 35 m³/h, feed rate 5 ml/min, atomization pressure 180 kPa and nozzle diameter 800 µm, resulting in a particle size of 30-60 µm.

### Example 3: Cellulose II-binder (95: 5)

The procedure of Example 2 was used, but in this case 5% w/w of the binder was used.

### Example 4: Preparation of verapamil.HCl and spironolactone tablets with the novel excipient by direct compression

The novel excipient obtained by method 1 (agglomerated with talc or kaolin) and the drug was mixed in a V-blender for 15 minutes. Tablets from the novel excipient and spironolactone (poorly soluble drug) or verapamil (highly soluble drug) were prepared according to what is shown in Table 3. Tablets were produced on a tableting machine of 1 station using flat punches of half inch diameter weighing 500 mg. Powders were compressed at a compression force of 20 kN. The dissolution profile of the tablets was measured.

**Table 3. Formulation bases using the novel excipient by direct compression, wet granulation and dry granulation technologies**

| **Ingredient** | (%) |
|---|---|
| Agglomerate MCCII | 83.5 |
| Spironolactone | 16.5 |

| **Ingredient** | |
|---|---|
| Agglomerate MCCII | 89.5 |
| Verapamil.HCl (%) | 10.5 |

### Example 5: Preparation of verapamil.HCl and spironolactone tablets with the novel excipient by wet granulation

Tablets of the novel excipient and verapamil.HCl (highly soluble drug, 50 mg dose) or spironolactone (poorly soluble drug, 80 mg dose) were prepared by mixing the two components in a V-blender for 15 minutes followed by the addition of these components (Table 3) into an oscillating granulator equipped with a # 24 mesh. Subsequently water was added without the need to add binders until forming a wet pulp for 10 minutes. The granules produced were dried in a convection oven tray at 60°C until the humidity content was <5%. The material was then passed through a # 40 mesh. Subsequently, the granulate was tableted on an 8 stations Riddhi tableting machine equipped with flat-faced punches of 13mm diameter. Finally, the dissolution profile was determined.

### Example 6: Preparation of verapamil.HCl and spironolactone tablets with the novel excipient by dry granulation

A mixture of the novel excipient and verapamil.HCl or spironolactone in a V-blender for 15 minutes (Table 3) was prepared. The mixture was pre-compressed in an 8 stations tableting machine equipped with flat-faced punches of 13 mm diameter producing ingots of -1.5 g. These ingots were passed to an oscillating granulator equipped with a # 24 mesh and were re-compressed into the same tableting machine to produce 500 mg tablets. The dissolution profile was determined in-vitro.

### Example 7: Preparation of verapamil.HCl and spironolactone tablets with commercial excipients by direct compression

Commercial MCCI (Avicel® PH101 and Prosolv® 90) was mixed in a V-blender for 15 minutes (Table 4) with spironolactone (poorly soluble drug) or verapamil (highly soluble drug). Tablets were prepared using a tableting machine of 1 station using flat punches of half inch diameter weighing 500 mg. The powders were compressed at a compression force of 20 kN. The dissolution profile was then determined.

**Table 4. Formulations using commercial excipients by direct compression, wet granulation and dry granulation technologies**

| **Ingredient** | (%) |
|---|---|
| MCCII (Avicel® PH101, or Prosolv® 90) | 83.5 |
| Spironolactone | 16.5 |

| **Ingredient** | (%) |
|---|---|
| MCCII (Avicel® PH101, or Prosolv® 90) | 89.5 |
| Verapamil.HCl (%) | 10.5 |

### Example 8: Preparation of verapamil.HCl and spironolactone tablets with commercial excipients by wet granulation

Commercial MCCI tablets (prepared Avicel® PH101 and Prosolv® 90) and verapamil.HCl (highly soluble drug, 50 mg) or spironolactone (poorly soluble drug, 80 mg) were prepared by mixing both components in a V-blender for 15 minutes followed by the addition of these components in an oscillating granulator equipped with a # 24 mesh, then water without binders was added to form a wet pulp for 10 minutes (Table 4).

The granules produced were dried in a convection oven tray at 60°C until the humidity content was <5%. The material was then passed through a # 40 mesh. Subsequently, the granulate was tableted on an 8 stations Riddhi tableting machine equipped with flat-faced punches of 13mm diameter. The dissolution profile was then determined in-vitro.

### Example 9: Preparation of verapamil.HCl and spironolactone tablets with commercial excipients by dry granulation

A mixture of commercial MCCI (Avicel® PH101 and Prosolv® 90) and verapamil.HCl or spironolactone was prepared in V-blender for 15 minutes (Table 4). The mixture was pre-compressed in an 8 stations tableting machine equipped with flat-faced punches of 13 mm diameter producing ingots of -1.5 g. These ingots were passed to an oscillating granulator equipped with a # 24 mesh and were re-compressed in the same tableting machine to produce 500 mg tablets. Finally, the dissolution profile was determined in-vitro.

### Example 10: Studies of in-vitro dissolution of verapamil.HCl tablets (80 mg dose)

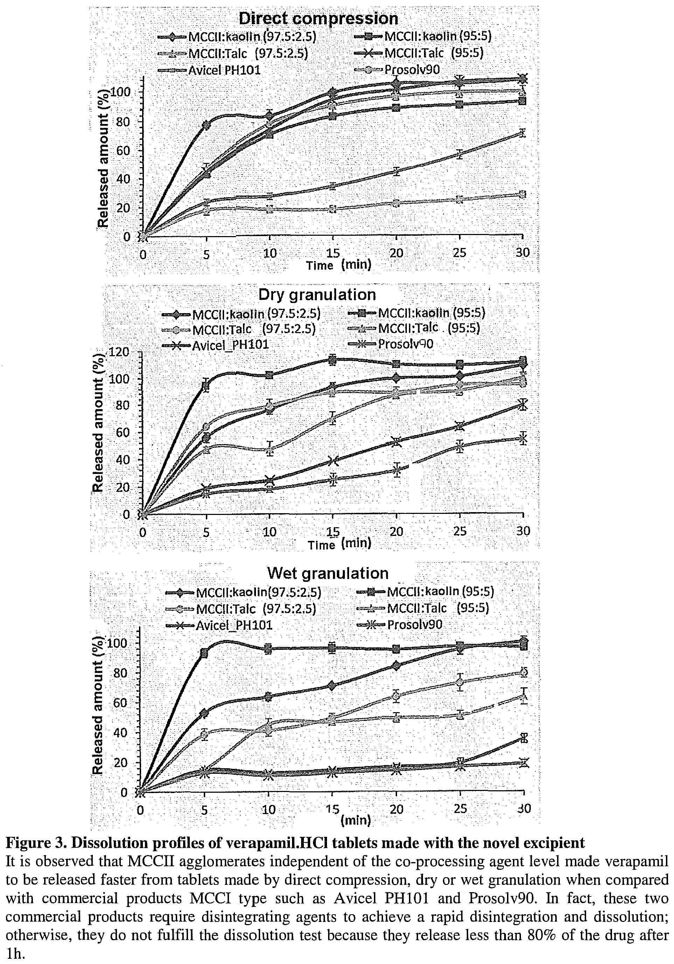

The study was performed in an Erweka DT8 dissolver apparatus II type equipped with 6 dissolution vessels, each with 900 mL of solvent and operated at 50 rpm (Fig. 3). The dissolution solution was HCl at 0.01 N. At 5, 10, 15, 20, 25 and 30 min, 1 mL aliquots were taken and diluted by half and the verapamil.HCl content was determined by UV spectrophotometric analysis at 278 nm.

### Example 11: Studies of in-vitro dissolution of spironolactone tablets (50 mg dose)

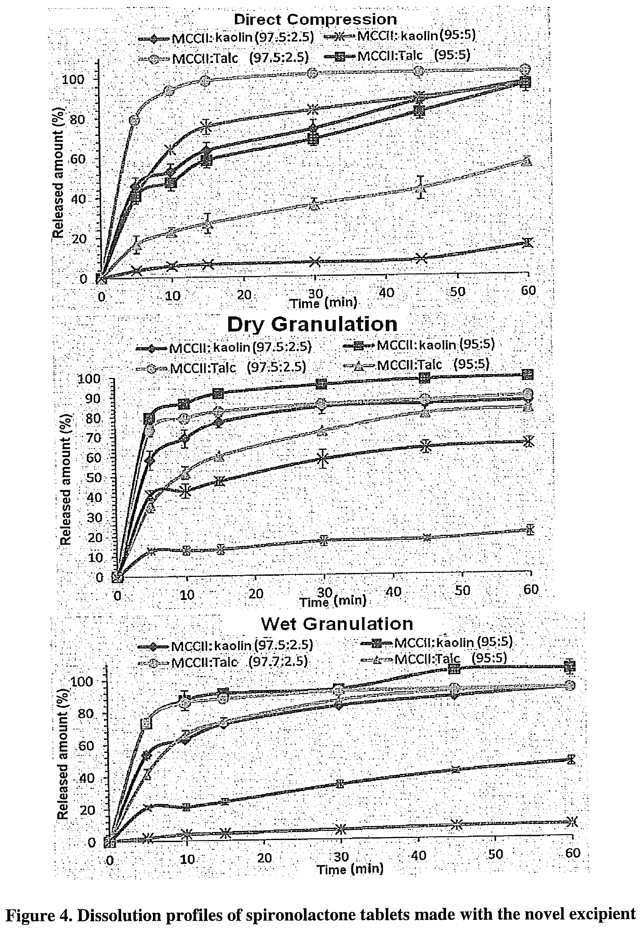

The study was performed in an Erweka DT8 dissolver apparatus II type equipped with 6 dissolution vessels, each with 1000 mL of solvent and 75 rpm (Fig. 4). The dissolution solution was HCl at 0.1 N with sodium lauryl sulfate at 0.1%. At 5, 10, 15, 30, 45 and 60 min, 1 mL aliquots were taken and diluted by two portions of the dissolution mean and the spironolactone content was determined by UV spectrophotometric analysis at 242 nm.

### Example 12: Preparation of a pattern I compact powder

The ingredients of Table 5 were taken and passed through a # 60 mesh. Then a binder dispersion (5-10%) was added and the mixture was manually mixed until wet and then added to a compacting machine where it was compressed at 200 pounds-force with a time of consolidation of 1 second.

**Table 5. Formulation of a pattern I compact powder**

| **Ingredient** | **Quantity (%w/w)** |
|---|---|
| Talc | 59 |
| Kaolin | 12 |
| Calcium carbonate | 12 |
| Titanium dioxide | 12 |
| Zinc stearate | 4 |
| Pigment (iron oxide, brown, black and yellow) | 1 |
| Binder | qs |
| Fragance | qs |

| **Ingredient** | **Quantity (%w/w)** |
|---|---|
| Tragacanth | 2 |
| Sorbitol | 5 |
| Water | 82.2 |
| Methyl paraben | 0.2 |
| Glyceryl monostearate | 0.6 |
| Mineral oil | 4 |

### Example 13: Preparation of compact powders with the novel agglomerated excipient

The agglomerate MCCII excipient was taken and mixed with ferric pigments to give the desired shade (Table 10). Then it was added to a compacting machine and compressed at 200 pounds-force with a consolidation time of 1 second without the need to add any binder.

**Table 6. Formulation of a compact powder with the novel agglomerated excipient**

| **Ingredient** | **Quantity (%w/w)** |
|---|---|
| Agglomerate MCCII | 99.3 |
| Brown pigment | 0.29 |
| Black pigment | 0.16 |
| Yellow pigment | 0.29 |

### Example 12: Preparation of facial loose powders with the novel agglomerated excipient

A direct mix of 99.5% agglomerated excipient and 0.5% fragrance and color was performed in a V-blender for 15 minutes and passed through a # 400 mesh prior packaging.

### Example 13: Preparation of body talc with the novel agglomerated excipient

A direct mix of 99.5% agglomerated excipient, 0.2% irgasan and 0.3% fragrance and color was performed in a V-blender for 15 minutes and passed through a # 400 mesh prior packaging.

### Example 14: Preparation of a body talc pattern

The ingredients listed in Table 7 were taken, mixed in a V-blender for 15 minutes and passed through a # 400 mesh prior packaging.

**Table 7. Formulation of a body talc pattern**

| **Ingredient** | **Quantity (%w/w)** |
|---|---|
| Talc | 67.8 |
| Kaolin | 10 |
| Magnesium stearate | 2 |
| Magnesium carbonate | 18 |
| Zinc stearate | 2 |
| Irgasan | 0.2 |
| Fragrance and color | qs |

### Example 18: Preparation of semisolid forms with the novel excipient

The ingredients listed in below Table 8 were taken and mixed to form aqueous dispersions with a homogenizer at 10,000 rpm for 10 min. Then the viscosity was measured to determine the effect of the plasticizer on the viscosity of the dispersion of cellulose II.

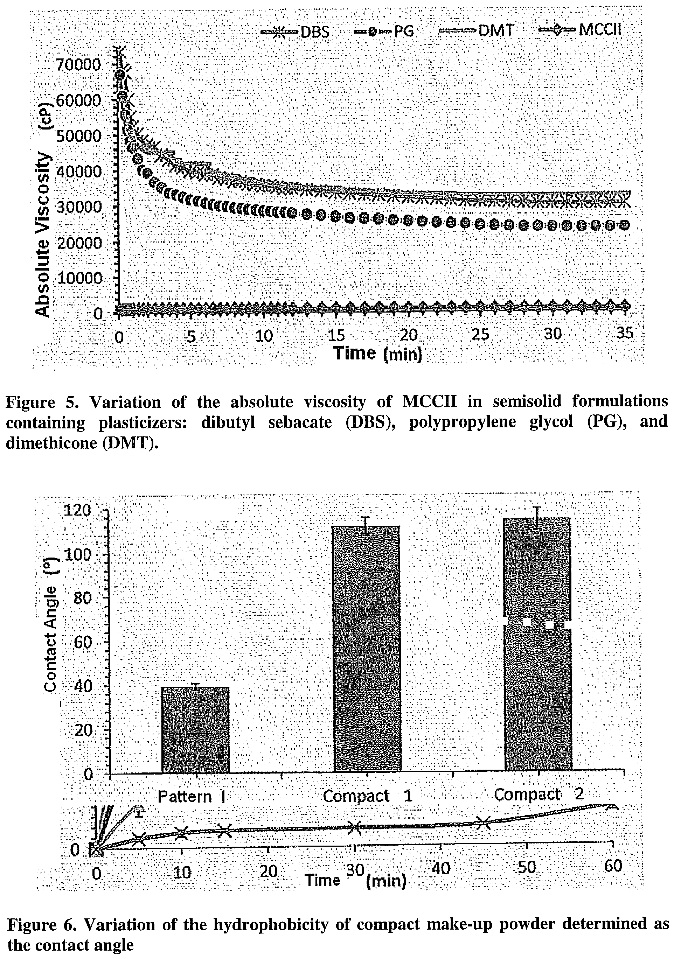

Figure 5 shows the variation of the viscosity of the novel excipient with some commercial plasticizers. It is noted that the viscosity greatly increases (∼ 20 times) in the presence of plasticizer (e.g., propylene glycol, dibutyl sebacate, dimethicone). This is very favorable when stable semisolids of high viscosity are needed.

### Example 19: Preparation a supplement for dietary fiber

About 80 g of MCCII crude fiber were taken and mixed with 10 g of wheat bran, 9.8 g of psyllium mucilagum and 0.2 of vitamin C and complex B (thiamine and riboflavin) in a V-blender for 10 min. Then the mixture was passed through a sieve with a #100 mesh and packed into airtight seal amber flasks. The dosage of this supplement is 30 g per day taken with meals dissolved in juice or water.

Figure 6 shows the contact angle of a pattern I compact powder relative to the compact powders I and II made with the novel agglomerated excipient. The results show that the hydrophobicity of the latter is high, resulting in a good adhesion on the skin, being better than the pattern I compact powder.

## Claims

1. An agglomerated cellulose excipient obtainable by a process comprising
1) obtaining a cellulose II crystalline form by a process comprising the following steps:
a. immersing hydrocellulose in an aqueous solution of sodium hydroxide in a ratio of 1:10, in an inert atmosphere;
b. cooling the solution to a temperature within -10°C and 10°C;
c. stirring the solution;
d. diluting the cellulose obtained in step c), for obtaining a gel with a concentration of 5% to 10% w/v of cellulose;
e. adding from 2.5% to 50% of binder, stirring at a rate of 10,000 rpm to 20,000 rpm for a period of 10-15 min;
f. neutralizing the solution from step e) with an organic acid selected from hydrochloric acid, nitric acid, sulfuric acid or mixtures thereof for obtaining a conductivity lower than 20 µS/cm;
g. optionally drying the dispersion by spray drying; or
h. optionally filtering the dispersion for obtaining a pulp with a solid contents within the range 1% - 60%;
i. granulating the product of step g) or step h) by dry or wet granulation; and
2) combining the cellulose of step 1 with a binder selected from talc, magnesium carbonate, amorphous silicon dioxide, bentonite, mica, titanium dioxide, zinc dioxide, kaolin, zinc stearate, chitosan, shellac, methacrylic acid derivatives or mixtures thereof.

2. The agglomerated cellulose excipient according to claim 1, **characterized in that** the hydrocellulose is selected from the group consisting of commercial cellulose and cellulose extracted from plant sources selected from cotton, sugar cane bagasse, sisal, rice hulls or corncobs or mixtures thereof.

3. The agglomerated cellulose excipient according to claim 2, **characterized in that** the cellulose is derived from cotton.

4. The agglomerated cellulose excipient according to claim 3, **characterized in that** prior to step a) a pre-treatment of cotton consisting of the following steps is performed:
a. immersing the cotton fibres in a dilute mineral acid;
b. heating the solution to a temperature within the range 100°C - 105°C for a maximum period of 3 hours.

5. The agglomerated cellulose excipient according to claim 4, **characterized in that** the mineral acid is selected from the group consisting of hydrochloric acid, sulfuric acid or nitric acid.

6. The agglomerated cellulose excipient according to claim 4, **characterized in that** the dilute acid is at a concentration within the range 0.5 N - 2.5 N.

7. The agglomerated cellulose excipient according to any of claims 4 to 6, **characterized in that** the mineral acid is hydrochloric acid at a concentration of 1.5 N.

8. The agglomerated cellulose excipient according to claim 4, **characterized in that** the pulp obtained at the end of the pre-treatment has solid concentrations within the range 1% - 60%.

9. The agglomerated cellulose excipient according to claim 8, **characterized in that** solid concentrations are within the range 10% - 30%.

10. The agglomerated cellulose excipient according to claim 2, **characterized in that** if the cellulose is obtained from sugar cane bagasse, sisal, rice hulls or corncobs, or mixtures thereof, these are subjected to a delignification process prior to step a) comprising the following steps:
a. combining sugar cane bagasse, sisal, rice hulls, corncobs or mixtures thereof with sodium hydroxide;
b. heating the mixture of step a) up to 80°C for a period of 3 hours;
c. washing, filtering and drying the material resulting from step b);
d. combining the material from step c) with sodium hypochlorite at room temperature for a period of 6-24 hours.

11. A process for manufacturing an agglomerated cellulose excipient, which comprises:
1) obtaining a cellulose II crystalline form by a process comprising the following steps:
a. immersing hydrocellulose in an aqueous solution of sodium hydroxide in a ratio of 1:10, in an inert atmosphere;
b. cooling the solution to a temperature within -10°C and 10°C;
c. stirring the solution;
d. diluting the cellulose obtained in step c), for obtaining a gel with a concentration of 5% to 10% w/v of cellulose;
e. adding from 2.5% to 50% of binder, stirring at a rate of 10,000 rpm to 20,000 rpm for a period of 10-15 min;
f. neutralizing the solution from step e) with an organic acid selected from hydrochloric acid, nitric acid, sulfuric acid or mixtures thereof for obtaining a conductivity lower than 20 µS/cm;
g. optionally drying the dispersion by spray drying; or
h. optionally filtering the dispersion for obtaining a pulp with a solid contents within the range 1% - 60%;
i. granulating the product of step g) or step h) by dry or wet granulation; and
2) combining the cellulose of step 1) with a binder selected from talc, magnesium carbonate, amorphous silicon dioxide, bentonite, mica, titanium dioxide, zinc dioxide, kaolin, zinc stearate, chitosan, shellac, methacrylic acid derivatives or mixtures thereof.

12. Use of the excipient of any one of claims 1 to 10 in the manufacturing of solid dosage forms of active compounds, **characterized in that** the said solid forms are selected from tablets, capsules or powders.

13. Use of the excipient of any one of claims 1 to 10 in the manufacturing of cosmetics, **characterized in that** said cosmetic products are selected from powders and bases.

14. Use according to claim 13, **characterized in that** the manufacturing comprises adding other pharmaceutically acceptable excipients and adjuvants.

15. Use according to claim 12, **characterized in that** the active compound is selected from drugs, food additives or agrochemicals.

## Patentansprüche

1. Agglomerierter Cellulose-Hilfsstoff, der durch ein Verfahren erhalten werden kann, das umfasst:
1) Erhalten einer kristallinen Cellulose-II-Form durch ein Verfahren, das die folgenden Schritte umfasst:
a. Eintauchen von Hydrocellulose in eine wässrige Lösung aus Natriumhydroxid in einem Verhältnis von 1:10 in einer inerten Atmosphäre,
b. Abkühlen der Lösung auf eine Temperatur innerhalb von -10 °C und 10 °C,
c. Rühren der Lösung,
d. Verdünnen der durch Schritt c) erhaltenen Cellulose, um ein Gel mit einer Konzentration von 5 Gew./Vol-% bis 10 Gew./Vol-% Cellulose zu erhalten,
e. Hinzufügen von 2,5 % bis 50 % Bindemittel, Rühren bei einer Rate von 10.000 U/min bis 20.000 U/min für einen Zeitraum von 10 -15 min,
f. Neutralisieren der Lösung aus Schritt e) mit einer organischen Säure, die aus Hydrochlorsäure, Salpetersäure, Schwefelsäure oder Mischungen davon ausgewählt ist, um eine Leitfähigkeit von weniger als 20 µS/cm zu erhalten,
g. optional Trocknen der Dispersion durch Sprühtrocknen, oder
h. optional Filtern der Dispersion, um einen Brei mit einem Feststoffgehalt im Bereich von 1 % - 60 % zu erhalten,
i. Granulieren des Produkts aus Schritt g) oder Schritt h) durch Trocken- oder Nassgranulation, und
2) Kombinieren der Cellulose von Schritt 1 mit einem Bindemittel, das aus Talk, Magnesiumcarbonat, amorphem Siliziumdioxid, Bentonit, Glimmer, Titandioxid, Zinkdioxid, Kaolin, Zinkstearat, Chitosan, Schellack, Methacrylsäurederivaten oder Mischungen davon ausgewählt ist.

2. Agglomerierter Cellulose-Hilfsstoff nach Anspruch 1, **dadurch gekennzeichnet, dass** die Hydrocellulose aus der Gruppe ausgewählt ist, die aus kommerzieller Cellulose und Cellulose besteht, die aus Pflanzenquellen extrahiert ist, welche aus Baumwolle, Zuckerrohrbagasse, Sisal, Reisschalen oder Maiskolben oder Mischungen davon ausgewählt sind.

3. Agglomerierter Cellulose-Hilfsstoff nach Anspruch 2, **dadurch gekennzeichnet, dass** die Cellulose von Baumwolle stammt.

4. Agglomerierter Cellulose-Hilfsstoff nach Anspruch 3, **dadurch gekennzeichnet, dass** vor Schritt a) eine Vorbehandlung von Baumwolle bestehend aus den folgenden Schritten durchgeführt wird:
a. Eintauchen der Baumwollfasern in eine verdünnte Mineralsäure,
b. Erwärmen der Lösung auf eine Temperatur im Bereich von 100 °C - 105 °C für einen maximalen Zeitraum von 3 Stunden.

5. Agglomerierter Cellulose-Hilfsstoff nach Anspruch 4, **dadurch gekennzeichnet, dass** die Mineralsäure aus der Gruppe ausgewählt ist, die aus Hydrochlorsäure, Schwefelsäure oder Salpetersäure besteht.

6. Agglomerierter Cellulose-Hilfsstoff nach Anspruch 4, **dadurch gekennzeichnet, dass** die verdünnte Säure in einer Konzentration im Bereich von 0,5 N - 2,5 N vorliegt.

7. Agglomerierter Cellulose-Hilfsstoff nach einem der Ansprüche 4 bis 6, **dadurch gekennzeichnet, dass** die Mineralsäure Hydrochlorsäure in einer Konzentration von 1,5 N vorliegt.

8. Agglomerierter Cellulose-Hilfsstoff nach Anspruch 4, **dadurch gekennzeichnet, dass** der am Ende der Vorbehandlung erhaltene Brei feste Konzentrationen im Bereich von 1 % - 60 % aufweist.

9. Agglomerierter Cellulose-Hilfsstoff nach Anspruch 8, **dadurch gekennzeichnet, dass** die festen Konzentrationen im Bereich von 10 % - 30 % liegen.

10. Agglomerierter Cellulose-Hilfsstoff nach Anspruch 2, **dadurch gekennzeichnet, dass**, wenn die Cellulose aus Zuckerrohrbagasse, Sisal, Reisschalen oder Maiskolben oder Mischungen davon erhalten wird, diese vor Schritt a) einem Delignifizierungsverfahren ausgesetzt werden, das die folgenden Schritte umfasst:
a. Kombinieren von Zuckerrohrbagasse, Sisal, Reisschalen, Maiskolben oder Mischungen davon mit Natriumhydroxid,
b. Erwärmen der Mischung von Schritt a) auf bis zu 80 °C für einen Zeitraum von 3 Stunden,
c. Waschen, Filtern und Trocknen des aus Schritt b) resultierenden Materials,
d. Kombinieren des Materials aus Schritt c) mit Natriumhypochlorit bei Raumtemperatur für einen Zeitraum von 6-24 Stunden.

11. Verfahren zur Herstellung eines agglomerierten Cellulose-Hilfsstoffs, das umfasst:
1) Erhalten einer kristallinen Cellulose-II-Form durch ein Verfahren, das die folgenden Schritte umfasst:
a. Eintauchen von Hydrocellulose in eine wässrige Lösung aus Natriumhydroxid in einem Verhältnis von 1:10 in einer inerten Atmosphäre,
b. Abkühlen der Lösung auf eine Temperatur innerhalb von -10 °C und 10 °C,
c. Rühren der Lösung,
d. Verdünnen der durch Schritt c) erhaltenen Cellulose, um ein Gel mit einer Konzentration von 5 Gew./Vol-% bis 10 Gew./Vol-% Cellulose zu erhalten,
e. Hinzufügen von 2,5 % bis 50 % Bindemittel, Rühren bei einer Rate von 10.000 U/min bis 20.000 U/min für einen Zeitraum von 10 -15 min,
f. Neutralisieren der Lösung aus Schritt e) mit einer organischen Säure, die aus Hydrochlorsäure, Salpetersäure, Schwefelsäure oder Mischungen davon ausgewählt ist, um eine Leitfähigkeit von weniger als 20 µS/cm zu erhalten,
g. optional Trocknen der Dispersion durch Sprühtrocknen, oder
h. optional Filtern der Dispersion, um einen Brei mit einem Feststoffgehalt im Bereich von 1 % - 60 % zu erhalten,
i. Granulieren des Produkts von Schritt g) oder Schritt h) durch Trocken- oder Nassgranulation, und
2) Kombinieren der Cellulose von Schritt 1) mit einem Bindemittel, das aus Talk, Magnesiumcarbonat, amorphem Siliziumdioxid, Bentonit, Glimmer, Titandioxid, Zinkdioxid, Kaolin, Zinkstearat, Chitosan, Schellack, Methacrylsäurederivaten oder Mischungen davon ausgewählt ist.

12. Verwendung des Hilfsstoffs nach einem der Ansprüche 1 bis 10 bei der Herstellung von festen Dosierungsformen von aktiven Verbindungen, **dadurch gekennzeichnet, dass** die festen Formen aus Tabletten, Kapseln oder Pulvern ausgewählt sind.

13. Verwendung des Hilfsstoffs nach einem der Ansprüche 1 bis 10 bei der Herstellung von Kosmetika, **dadurch gekennzeichnet, dass** die Kosmetikprodukte aus Pulvern und Basen ausgewählt sind.

14. Verwendung nach Anspruch 13, **dadurch gekennzeichnet, dass** das Herstellen das Hinzufügen anderer pharmazeutisch annehmbarer Hilfsstoffe und Zusatzstoffe umfasst.

15. Verwendung nach Anspruch 12, **dadurch gekennzeichnet, dass** die aktive Verbindung aus Medikamenten, Lebensmittelzusatzstoffen oder Agrochemikalien ausgewählt ist.

## Revendications

1. Excipient en cellulose agglomérée susceptible d'être obtenu par un procédé comprenant :
1) l'obtention d'une forme cristalline II de cellulose par un procédé comprenant les étapes suivantes :
a. immersion d'hydrocellulose dans une solution aqueuse de soude dans un rapport 1:10, sous atmosphère inerte ;
b. refroidissement de la solution à une température entre -10°C et 10°C ;
c. agitation de la solution;
d. dilution de la cellulose obtenue à l'étape c), pour obtenir un gel ayant une concentration en cellulose de 5% à 10% m/v ;
e. addition de 2,5% à 50% de liant, agitation à une vitesse de 10 000 tr/min à 20 000 tr/min pendant une durée de 10-15 min ;
f. neutralisation de la solution de l'étape e) avec un acide organique choisi parmi l'acide chlorhydrique, l'acide nitrique, l'acide sulfurique et leurs mélanges pour obtenir une conductivité inférieure à 20 µS/cm ;
g. éventuellement séchage de la dispersion par atomisation; ou
h. éventuellement filtration de la dispersion pour obtenir une pulpe ayant une teneur en solides dans la gamme 1% - 60% ;
i. granulation par voie sèche ou par voie humide du produit de l'étape g) ou de l'étape h) ; et
2) la combinaison de la cellulose de l'étape 1 avec un liant choisi parmi le talc, le carbonate de magnésium, la silice amorphe, la bentonite, le mica, le dioxyde de titane, le dioxyde de zinc, le kaolin, le stéarate de zinc, le chitosan, la gomme-laque, les dérivés d'acide méthacrylique et leurs mélanges.

2. Excipient en cellulose agglomérée selon la revendication 1, **caractérisé en ce que** l'hydrocellulose est choisie dans le groupe constitué de cellulose commerciale et de cellulose extraite d'une source végétale choisie parmi le coton, la bagasse de canne à sucre le sisal l'écorce de riz, un épi de maïs, et leurs mélanges.

3. Excipient en cellulose agglomérée selon la revendication 2, **caractérisé en ce que** la cellulose est dérivée du coton.

4. Excipient en cellulose agglomérée selon la revendication 3, **caractérisé en ce que** préalablement à l'étape a) on effectue un pré-traitement du coton constitué des étapes suivantes :
a. l'immersion des fibres de coton dans un acide inorganique dilué ;
b. le chauffage de la solution à une température dans la gamme 100°C - 105°C pendant une durée maximum de 3 heures.

5. Excipient en cellulose agglomérée selon la revendication 4, **caractérisé en ce que** l'acide inorganique est choisi dans le groupe constitué de l'acide chlorhydrique, l'acide sulfurique et l'acide nitrique.

6. Excipient en cellulose agglomérée selon la revendication 4, **caractérisé en ce que** la concentration de l'acide dilué est dans la gamme 0,5 N - 2,5 N.

7. Excipient en cellulose agglomérée selon l'une des revendications 4 à 6, **caractérisé en ce que** l'acide inorganique est l'acide chlorhydrique à une concentration de 1,5 N.

8. Excipient en cellulose agglomérée selon la revendication 4, **caractérisé en ce que** la pulpe obtenue à l'issue du pré-traitement a une teneur en solides dans la gamme 1% - 60%.

9. Excipient en cellulose agglomérée selon la revendication 8, **caractérisé en ce que** la teneur en solides est dans la gamme 10% - 30%.

10. Excipient en cellulose agglomérée selon la revendication 2, **caractérisé en ce que** si la cellulose est obtenue à partir de la bagasse de canne à sucre, de sisal, d'écorce de riz, d'épi de maïs, ou de leurs mélanges, ces matières sont soumises à un procédé de délignification avant l'étape a), procédé comprenant les étapes suivantes :
a. combinaison de la bagasse de canne à sucre, du sisal, de l'écorce de riz, de l'épi de maïs, ou de leurs mélanges, avec de la soude ;
b. chauffage du mélange de l'étape a) jusqu'à 80°C pendant une durée de 3 heures ;
c. lavage, filtration et séchage du matériau résultant de l'étape b);
d. combinaison du matériau de l'étape c) avec de l'hypochlorite de sodium à température ambiante pendant une durée de 6-24 heures.

11. Procédé de fabrication d'un excipient en cellulose agglomérée, qui comprend :
1) l'obtention d'une forme cristalline II de cellulose par un procédé comprenant les étapes suivantes :
a. immersion d'hydrocellulose dans une solution aqueuse de soude dans un rapport 1:10, sous atmosphère inerte ;
b. refroidissement de la solution à une température entre -10°C et 10°C ;
c. agitation de la solution;
d. dilution de la cellulose obtenue à l'étape c), pour obtenir un gel ayant une concentration en cellulose de 5% à 10% m/v ;
e. addition de 2,5% à 50% de liant, agitation à une vitesse de 10 000 tr/min à 20 000 tr/min pendant une durée de 10-15 min ;
f. neutralisation de la solution de l'étape e) avec un acide organique choisi parmi l'acide chlorhydrique, l'acide nitrique, l'acide sulfurique et leurs mélanges pour obtenir une conductivité inférieure à 20 µS/cm ;
g. éventuellement séchage de la dispersion par atomisation; ou
h. éventuellement filtration de la dispersion pour obtenir une pulpe ayant une teneur en solides dans la gamme 1% - 60% ;
i. granulation par voie sèche ou par voie humide du produit de l'étape g) ou de l'étape h) ; et
2) la combinaison de la cellulose de l'étape 1 avec un liant choisi parmi le talc, le carbonate de magnésium, la silice amorphe, la bentonite, le mica, le dioxyde de titane, le dioxyde de zinc, le kaolin, le stéarate de zinc, le chitosan, la gomme-laque, les dérivés d'acide méthacrylique et leurs mélanges.

12. Utilisation de l'excipient selon l'une quelconque des revendications 1 à 10 dans la fabrication de formes de dosage solides de composés actifs, **caractérisée en ce que** lesdites formes solides sont choisies parmi les comprimés, les gélules et les poudres.

13. Utilisation de l'excipient selon l'une quelconque des revendications 1 à 10 dans la fabrication de produits cosmétiques, **caractérisée en ce que** lesdits produits cosmétiques sont choisis parmi les poudres et les bases.

14. Utilisation selon la revendication 13, **caractérisée en ce que** la fabrication comprend l'addition d'autres excipients et adjuvants pharmaceutiquement acceptables.

15. Utilisation selon la revendication 12, **caractérisée en ce que** le compose actif est choisi parmi les médicaments, les additifs alimentaires et les produits agrochimiques.
